**Europäisches Patentamt**

**European Patent Office**

**Office européen des brevets**

(11) Veröffentlichungsnummer: **0 382 808 B1**

(12) **EUROPÄISCHE PATENTSCHRIFT**

(45) Veröffentlichungstag der Patentschrift: **12.10.94**

(51) Int. Cl.⁵: **A61B 3/10**, G02B 3/02

(21) Anmeldenummer: **89907090.8**

(22) Anmeldetag: **29.06.89**

(86) Internationale Anmeldenummer:
**PCT/DE89/00434**

(87) Internationale Veröffentlichungsnummer:
**WO 90/00026 (11.01.90 90/02)**

(54) **VORRICHTUNG ZUR ERZEUGUNG EINES BILDES EINES OBJEKTS.**

(30) Priorität: **29.06.88 DE 3821973**

(43) Veröffentlichungstag der Anmeldung:
**22.08.90 Patentblatt 90/34**

(45) Bekanntmachung des Hinweises auf die
Patenterteilung:
**12.10.94 Patentblatt 94/41**

(84) Benannte Vertragsstaaten:
**AT CH DE FR LI**

(56) Entgegenhaltungen:
**EP-A- 0 161 645**
**WO-A-83/02717**
**DE-C- 0 587 079**
**US-A- 4 213 678**

(73) Patentinhaber: **G. RODENSTOCK INSTRUMEN-
TE GMBH**
**Otto-Hahn-Strasse 11**
**D-85521 Ottobrunn (DE)**

(72) Erfinder: **TRILLER, Adolf**
**Regerstr. 23**
**D-8032 Lochham (DE)**

(74) Vertreter: **Münich, Wilhelm, Dr. et al**
**Kanzlei Münich, Steinmann, Schiller**
**Wilhelm-Mayr-Str. 11**
**D-80689 München (DE)**

## Beschreibung

### Technisches Gebiet

Die Erfindung bezieht sich auf eine Vorrichtung zur Erzeugung eines Bildes eines Objekts und insbesondere zur Beobachtung des Auges gemäß dem Oberbegriff des Patentanspruchs 1.

### Stand der Technik

Bei der Beobachtung der hinteren Augenabschnitte besteht die Schwierigkeit, daß die Beleuchtung und die Beobachtung durch die Augenpupille und die häufig optisch nicht klaren vorderen Augenmedien erfolgen muß, an denen Reflexe auftreten, und die Abbildungsfehler erzeugen.

Es ist deshalb bereits seit längerem vorgeschlagen worden, zur Beobachtung der hinteren Augenabschnitte anstelle von konventionellen Funduskameras scannende bzw. abtastende Vorrichtungen zu verwenden, bei denen der Augenhintergrund nicht großflächig ausgeleuchtet wird, sondern die mit einem auf einem möglichst kleinen Fleck fokussierten Beleuchtungslichtstrahl den Augenhintergrund abtasten und das reflektierte Licht in Zuordnung zur Abtastsequenz erfassen. Hierzu wird beispielsweise auf "The Foundations of Ophtalmology, Band 7, S. 307/308, Jahrgang 1962, die US-PS 4 213 678, die japanischen Patentveröffentlichungen JP-B-61-5730 und JP-A-50-138822 sowie die EP-A-0 145 563 verwiesen.

Die bekannten Einrichtung zur Beobachtung des Auges haben damit eine Reihe von Nachteilen: Durch die Verkippung der Elemente, die den Abtastlichtstrahl und den reflektierten bzw. gestreuten Lichtstrahl abbilden, gegeneinander, sowie durch die zweidimensionale strahlablenkung, die für die x/y-Abtastbewegung erforderlich ist, ist der Abbildungsmaßstab über die Bildfläche nicht konstant.

Dies führt dazu, daß ein Rechteck trapezförmig verzerrt wird. Dies ist insbesondere dann störend, wenn beispielsweise bei einer elektronischen Bildauswertung nacheinander aufgenommene Bereiche "aneinander angesetzt" werden sollen.

### Beschreibung der Erfindung

Der Erfindung liegt die Aufgabe zugrunde, eine Vorrichtung zur Gewinnung eines Bildes und insbesondere zur Beobachtung des Auges gemäß dem Oberbegriff des Patentanspruchs 1 derart weiterzubilden, daß die trapezförmige Verzeichung kompensiert ist.

Eine erfindungsgemäße Lösung dieser Aufgabe ist mit ihren Weiterbildungen in den Patentansprüchen angegeben.

Erfindungsgemäß wird diese Aufgabe dadurch gelöst, daß im Strahlengang zwischen den Abtastelementen und dem zu abzubildenden Objekt wenigstens ein asphärisches Elemente vorgesehen ist, das die trapezförmige Verzeichnung kompensiert.

Bei einer Weiterbildung der Erfindung ist zusätzlich ein zweites asphärisches Element vorgesehen, das das Bild in der Pupillenebene des Auges einschnürt; das erste asphärische Element kompensiert dann die Trapez-Verzeichnung in der Ebene des zweiten Elements.

Beispielsweise bei ophthalmologischen Anwendungen wird das Strahlenbündel in der Regel in der Augenpupille eingeschnürt. Da diese strahleinschnürung des Pupillenbildes beachtet werden muß, ist es vorteilhaft, ein zweites asphärisches Element vorzusehen, das einen zusätzlichen Freiheitsgrad bei der Korrektion gibt.

Mit dem ersten Element wird eine Entzerrung in der Ebene des zweiten Elements herbeigeführt. Das zweite Element sorgt dann für eine exakte Strahleinschnürung im Pupillenbild.

Wenn sowohl die Entzerrung im Pupillenbild als auch die Strahleinschnürung in der Pupillenebene erfüllt ist, so sind auch alle weiteren ebenen Schnittbilder durch das Strahlenbündel entzerrt.

Weiterhin ist es bevorzugt, wenn die Brechkraft der beiden asphärischen Elemente im Bereich der optischen Achse annähernd Null ist.

### Kurze Beschreibung der Zeichnung

Die Erfindung wird nachstehend anhand eines Ausführungsbeispiels unter Bezugnahme auf die Zeichnung näher beschrieben, in der zeigen:

Fig. 1     einen Schnitt durch einen Teil einer erfindungsgemäßen Vorrichtung zeigt, und

Fig. 2     schematisch eine Korrektionsfläche.

### Beschreibung eines Ausführungsbeispiels

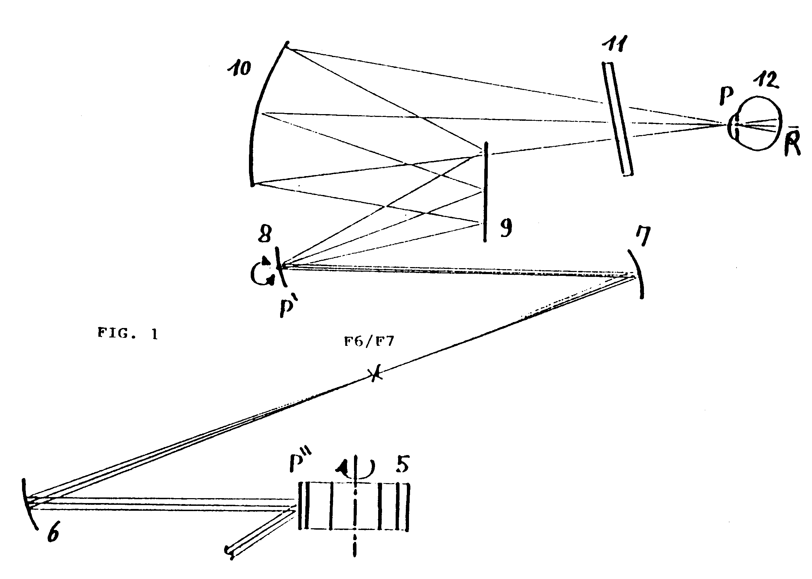

Die in Fig. 1 ausschnittsweise dargestellte erfindungsgemäße Vorrichtung weist eine nicht dargestellte Beleuchtungs-Lichtquelle, beispielsweise einen Laser, sowie eine nicht dargestellte Detektoreinrichtung auf, deren Ausgangssignal von einer Auswerte- und Synchronisiereinheit ausgewertet und beispielsweise auf einem Monitor dargestellt wird. Bei dem gezeigten Ausführungsbeispiel "verlaufen" sowohl der Beleuchtungslichtstrahl als auch der vom Augenhintergrund kommende Lichtstrahl über die Ablenkeinrichtung.

Der Lichtstrahl des Lasers wird von einem ersten Ablenkelement (Horizontal-Scanner), der bei dem gezeigten Ausführungsbeispiel ein sich drehender Polygonspiegel 5 ist, in Horizontalrichtung (senkrecht zur Zeichenebene) abgelenkt. Der nun in der Horizontalebene aufgefächerte Strahl durchläuft das Spiegelsystem 6 und 7, und trifft auf ein zweites Ablenkelement (Vertikal-Scanner), der bei dem gezeigten Ausführungsbeispiel ein schwing- bzw. Galvanometerspiegel 8 ist, auf. Nach dem Spiegel 8 hat das Strahlbündel einen "rechteckigen" Querschnitt. Nach Umlenkung an einem weiter unten noch beschriebenen Spiegel 9 wird er von einem Konkavspiegel 10 über ein weiter unten noch beschriebenes Element 11 auf das zu untersuchende Auge 12 abgebildet. Der reflektierte Lichtstrahl durchläuft in umgekehrter Reihenfolge die genannten Elemente und wird nach dem Horizontal-Ablenkelement 5 von einem nicht dargestellten Detektor nach vorheriger Trennung des Beleuchtungs- und des Beobachtungslichtwegs nachgewiesen.

Erfindungsgemäß ist nun erkannt worden, daß sich bei einer Vorrichtung gemäß Fig. 1 eine trapezförmige Verzeichnung ergibt. Diese trapezförmige Verzeichnung wird dadurch korrigiert, daß die Elemente 9 und 11 erfindungsgemäß ausgestaltet sind. Dies soll im folgenden beschrieben werden.

Hierzu wird mit dem ersten Element, beispielsweise dem Element 9 eine Entzerrung der Trapez-Verzeichnung in der Ebene des zweiten Elements, also beispielsweise des Elements 11 herbeigeführt. Das zweite Element sorgt dann für eine exakte strahleinschnürung im Pupillenbild P.

Wenn sowohl die Entzerrung im Pupillenbild als auch die Strahleinschnürung in der Pupillenebene erfüllt ist, so sind auch alle weiteren ebenen Schnittbilder durch das Strahlenbündel entzerrt.

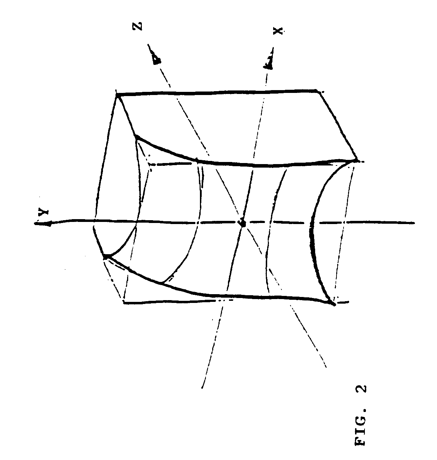

Im Falle der in Fig.1 dargestellten näherungsweisen 1:1 Abbildung der Pupille ist es bevorzugt, wenn die Elemente 9 bzw. 11 asphärische Flächen aufweisen, deren optische Wirkung in der Mitte, d.h. im Bereich der optischen Achse annähernd gleich Null ist. Von diesem Bezugspunkt ausgehend, gehen die Flächen längs der Reflexionsebene des Hohlspiegels nach der einen Seite in eine konvexe, nach der anderen Seite in eine konkave Krümmung über, deren Größe auf die erforderliche Entzerrung abgestimmt ist. Dies zeigt schematisch Fig. 2, der auch das im folgenden verwendete Koordinatensystem x,y,z zu entnehmen ist.

Die Fläche kann beispielsweise der Funktion F

$$F \equiv \Gamma^*(x^2 + z^2) - 2z = 0$$

gehorchen, wobei $\Gamma$ der Kehrwert des Radius der Querbögen Q ist und von y abhängig ist. Die Koordinaten liegen in der Reflexionsebene des Elements 9. Bei der folgenden Abhängigkeit der Krümmung $\Gamma$ von y:

$$\Gamma = c_1 y + c_2 y^2 + c_3 y^3$$

ergibt sich beispielsweise folgende Flächenfunktion:

$$F \equiv (c_1 y + c_2 y^2 + c_3 y^3)^*(x^2 + z^2) - 2z = 0$$

Bei den vorstehenden Gleichungen liegt die Verbindungslinie der Scheitel der Querbögen Q auf der y-Achse. Führt man auch hier eine Krümmung ein, die zweckmäßiger Weise einer Kurve dritter ORdnung folgt, so erhält man als "erweiterte" Flächenfunktion:

$$F \equiv (c_1 y + c_2 y^2 + c_3 y^3)^*(x^2 + z^2 - 2zy^3 c_4) - 2z + 2y^3 c_4 = 0$$

Über die Konstante $c_4$ kann die Krümmung der Verbindungslinie der Scheitel gesteuert werden.

Die vorstehend angeführten exemplarischen Gleichung gelten auch für das Element 11, das eine planparallele Platte sein kann, deren eine "ebene Fläche" beispielsweise entsprechend den vorstehenden Gleichungen "asphärisch modifiziert" ist.

Durch entsprechende Wahl der Größen $c_i$, $i = 1,2,3,4$ lassen sich die vorstehend genannten Bedingungen - Entzerrung im Pupillenbild und exakte Strahleinschnürung in der Pupillenebene - erfüllen, so daß auch alle weiteren ebenen Schnittbilder durch das Strahlenbündel entzerrt sind.

Vorstehend ist die Erfindung anhand eines Ausführungsbeispiels ohne Beschränkung des allgemeinen Erfindungsgedankens und insbesondere ohne Beschränkung der allgemeinen Anwendbarkeit der vorstehend exemplarisch für ophthalmologische Anwendungen beschriebenen Vorrichtung beschrieben worden.

**Patentansprüche**

1.  Vorrichtung zur Erzeugung eines Bildes eines Objekts und insbesondere zur Beobachtung des Hintergrundes eines Auges (12), mit einer Beleuchtungslichtquelle, die einen Lichtstrahl erzeugt, der auf einen zu beobachtenden Objektabschnitt fokussiert wird, einer Abtasteinrichtung (5-8,10), die eine Abtastbewegung des Lichtstrahls der Beleuchtungslichtquelle über den zu beobachtenden Objektabschnitt erzeugt, wobei die Abtasteinrichtung die Abtastbewegung erzeugende Elemente (5,8) und zusätzlich abbildende Elemente (6,7,10) aufweist, einer Detektoreinrichtung, die das von dem zu beobachtenden Objektabschnitt über die Abtasteinrichtung reflektierte Licht empfängt und ein zeitsequentielles Ausgangssignal erzeugt, und

einer Auswerte- und Synchronisiereinheit, die aus dem zeitsequentiellen Ausgangssignal der Detektoreinrichtung ein Bild des Objektabschnitts erzeugt,
dadurch **gekennzeichnet**, daß im Strahlengang zwischen den Abtastelementen und dem zu beobachtenden Objekt abschnitt wenigstens ein asphärisches Element (9,10) vorgesehen ist, das sowohl durch die Entzerrung des Pupillenbildes als auch durch eine Strahleinschnürung in der Pupillenebene (P) der Abtasteinrichtung die trapezförmige Verzeichnung kompensiert.

2. Vorrichtung nach Anspruch 1,
dadurch **gekennzeichnet**, daß zusätzlich ein zweites asphärisches Element (11) vorgesehen ist, das das Bild in der Pupillenebene (P) des Auges (12) einschnürt, und
daß das erste asphärische Element (9) die Trapez-Verzeichnung in der Ebene des zweiten Elements (11) kompensiert.

3. Vorrichtung nach Anspruch 1 oder 2,
dadurch **gekennzeichnet**, daß die Brechkraft der beiden asphärischen Elemente (9,11) im Bereich ihrer optischen Achse annähernd Null ist.

4. Vorrichtung nach einem der Ansprüche 1 bis 3,
dadurch **gekennzeichnet**, daß das erste asphärische Element (9) ein Spiegel ist.

5. Vorrichtung nach einem der Ansprüche 1 bis 4,
dadurch **gekennzeichnet**, daß eines der abbildenden Elemente (6,7,10) ein Konkavspiegel ist, und daß die beiden asphärischen Elemente (9,11) im Strahlengang beidseits des Konkavspiegels (10) angeordnet sind.

6. Vorrichtung nach Anspruch 5,
dadurch **gekennzeichnet**, daß die Abstände der asphärischen Elemente (9,11) zum Konkavspiegel (10) näherungsweise gleich sind.

## Claims

1. Device for producing an image of an object and especially for observing the background of the eye (12), with an illuminating light source which produces a light beam which is focused onto an a section of an object to be observed, a scanning device (5-8, 10) which produces a scanning movement for the light beam of the illuminating light source over the section of the object to be observed, wherein the scanning device has elements (5, 8) for producing the scanning movement and additionally imaging elements (6, 7, 10),
a detector device which receives the light reflected by the scanning device from the section of the object to be observed and produces a time sequential output signal, and an evaluating and synchronising unit which from the time sequential output signal of the detector device produces an image of the section of the object, **characterised** in that in the beam path between the scanning elements and the section of the object to be observed there is provided at least one non-spherical element (9, 10) which compensates the trapezoidal distortion both through the restitution of the image of the pupil as well as through a beam constriction in the plane of the pupil (P) of the scanning device.

2. Device according to claim 1,
**characterised** in that additionally there is provided a second non-spherical element (11) which constricts the image in the plane of the pupil (P) of the eye (12), and that the first non-spherical element (9) compensates the trapezoidal distortion in the plane of the second element (11).

3. Device according to claim 1 or 2,
**characterised** in that the refractive power of the two non-spherical elements (9, 11) in the area of their optical axis is almost zero.

4. Device according to one of the claims 1 to 3,
**characterised** in that the first non-spherical element (9) is a mirror.

5. Device according to one of the claims 1 to 4,
**characterised** in that one of the imaging elements (6, 7, 10) is a concave mirror and that the two non-spherical elements (9, 11) are arranged in the beam path on both sides of the concave mirror (10).

6. Device according to claim 5,
**characterised** in that the distances of the non-spherical elements (9, 11) from the concave mirror (10) are approximately the same.

## Revendications

1. Dispositif pour produire une image d'un objet et en particulier pour observer un fond d'oeil (12), ce dispositif comportant une Source (2) de lumière d'éclairage, qui produit un rayon lumineux qui est focalisé sur une tranche d'objet à observer, un dispositif d'exploration (5-8, 10), qui provoque un mouvement d'exploration réalisé par le rayon lumineux de la source de lumière d'éclairage sur la tranche d'objet à observer, le dispositif d'observation présentant des éléments (5, 8) produisant le mouvement d'exploration et, en outre, des éléments (6, 7, 10) de formation d'image, un dispositif détecteur qui reçoit la lumière, provenant de la tranche d'objet à observer et réfléchie par l'intermédiaire du dispositif d'exploration, et produit successivement dans le temps un signal de sortie, et une unité d'exploitation et de synchronisation qui, à partir du signal de sortie produit successivement dans le temps par le dispositif détecteur fournit une image de la tranche de l'objet, dispositif caractérisé en ce que, sur le trajet des rayons entre les éléments d'exploration et la tranche d'objet à observer, il y a au moins un élément asphérique (9, 10) qui, aussi bien par le redressement de l'image de la pupille que, également, par un resserrement du rayon dans le plan de la pupille (P) du dispositif d'exploration compense la distorsion trapézoïdale.

2. Dispositif selon la revendication 1 caractérisé en ce qu'il comporte en plus un second élément asphérique (11), qui resserre l'image dans le plan de la pupille (P) de l'oeil (12) et en ce que le premier élément asphérique (9) compense la distorsion trapézoïdale dans le plan du second élément (11).

3. Dispositif selon la revendication 1 ou 2, caractérisé en ce que le pouvoir de réfraction des deux éléments asphériques (9, 11) est approximativement nul dans la zone de leur axe optique.

4. Dispositif selon l'une, des revendications 1 à 3, caractérisé en ce que le premier élément asphérique (9) est un miroir.

5. Dispositif selon l'une des revendications 1 à 4, caractérisé en ce que l'un des éléments (6, 7, 10) formateur d'image est un miroir concave et en ce que les deux éléments asphériques (9, 11) sont disposés, sur le trajet des rayons, des deux côtés du miroir concave (10).

6. Dispositif selon la revendication 5, caractérisé en ce que les distances des éléments asphériques (9, 11) par rapport au miroir concave (10) sont approximativement égales.

FIG. 1

FIG. 2